# EUROPEAN PATENT APPLICATION

(11) **EP 4 556 990 A1**
(43) Date of publication of application: **21.05.2025**
(21) Application number: 23210417.4
(22) Date of filing: 16.11.2023
(51) Int. Cl.: G02C 7/08, A61F 9/00, G02C 7/10, G02C 11/04, G02C 11/00, G02C 5/00

(54) **SYSTEM, METHOD, AND COMPUTER PROGRAM FOR CONTROLLING A PROGRESSION OF MYOPIA IN AT LEAST ONE EYE OF A PERSON**

(71) Applicant: Carl Zeiss Vision International GmbH, 73430 Aalen (DE)
(72) Inventor: Barraza-Bernal, Maria Jose, 73431 Aalen (DE); Habtegiorgis, Selam Wondimu, 73431 Aalen (DE); Jester, Philipp, 89520 Heidenheim (DE)
(74) Representative: Altmann Stößel Dick Patentanwälte PartG mbB

(57) **Abstract**

The present invention relates to a system (110), a method, and a computer program for controlling a progression of myopia in at least one eye of a person, wherein the system comprises:
- a spectacle frame (112) comprising a pair of spectacle lenses (114, 114') and at least one of the spectacle lenses (114, 114') incorporated by the spectacle frame (112), wherein the spectacle frame (114) or at least one of the spectacle lenses (114, 114') are configured for applying a treatment process during a period of time, wherein the treatment process is designed for reducing the progression of myopia in at least one eye of a person, wherein the treatment process is determined by at least one characteristic parameter, wherein the treatment process is selected from a class of treatment processes comprising:
o an optical treatment of the at least one eye of the person;
o a radiation treatment of the at least one eye of the person;
o a pharmaceutical treatment of the at least one eye of the person; and
o a behavioral treatment of the person,
and
- a processing device (116) configured for determining at least one result of the treatment process on the progression of the myopia in the at least one eye of the person, wherein the spectacle frame (112) or at least one of the spectacle lenses (114, 114') are configured for applying at least two treatment processes, each selected from a different class, simultaneously to the person during a first period of time (210).

## Description

### Field of the invention

The present invention relates to a system, a method, and a computer program for controlling a progression of myopia in at least one eye of a person.

### Related art

According to Németh et al., 2021, Update and guidance on management of myopia. European Society of Ophthalmology in cooperation with International Myopia Institute, European Journal of Ophthalmology 31.3 (2021):853-883, myopia is a global leading ocular condition and with an incidence increasing in accelerated pace is considered as the most common cause of irreversible visual impairments.

As described by Fricke TR, Jong M, Naidoo KS, et al., Global prevalence of visual impairment associated with myopic macular degeneration and temporal trends from 2000 through 2050: systematic review, meta-analysis and modelling, Br J Ophthalmol. 2018; 102(7):855-862, the worldwide forecast for visual impairments associated with myopic macular degeneration shows alarming values for the year 2050. This burden will increase significantly when no measures are taken to decrease the development of myopia, reduce its progression and improve techniques for myopia management.

This situation rises a motivation to combine efforts to understand the basic mechanism underlying myopia development and to develop management tools that address its onset and progression. The cause for myopia and progression of myopia has been demonstrated to be multifactorial, wherein the following factors have been reported:
- peripheral signals, e.g. defocus, color, or contrast;
- a lag of accommodation;
- environmental, in particular a spectral composition of irradiation; and
- behavioral, e.g. an amount of time spent on near-vision working or outdoor activities.

Addressing behavior by means of time spent on outdoor activities seems to be an easy and not invasive alternative. Accordingly, behavioral solutions have been suggested as gadgets to control the time spent on near-vision working or outdoor activities:
CN105468147A discloses a device for preventing myopia, comprising a microprocessor, a wireless communication module, a camera, a plurality of sensors, an audio output interface, an alarm, a vibrator, a power supply management module, a chargeable battery, a wireless charging module and a wire charging module; a system based on the device comprising a cloud server, a personal mobile terminal and an authorizer mobile terminal; the device is respectively connected to the personal mobile terminal and the cloud server through wireless network; a method for preventing myopia based on the device and the system comprises a terminal and the personal mobile terminal carry out data interaction; the terminal and the cloud server carry out data interaction; the cloud server carries out big data analysis and provides alarm and pre-alarm. The invention can implement local alarm and remote alarm and sends the user information to the cloud server; the cloud server sends the user information to an authorization guardian; and the eye using behavior of the user is monitored in real time and is effectively adjusted by the authorized guardian.

CN110141185A discloses wearable equipment for preventing myopia and monitoring sight and having a visual training function. The wearable equipment comprises a microcomputer control system, a power source management system, a mechanical system, a voice system, an environment sensing system and an image system, wherein the microcomputer control system is used for receiving information fed back by each system, processing the information, and then transmitting control signals to the systems to complete corresponding functions; the mechanical system can replace a lens of a visual lens window of the wearable equipment; the voice system can send a sound prompt to a wearer and acquire answer information; and the image system can shoot high-definition images and perform fuzzification treatment. Through the contrast of naked eye imaging clarity with actual imaging clarity, the sight level of the wearer is reminded; through visual training, the wearable equipment can assist the wearer to exercise crystalline lenses, and help the wearer to prevent myopia and monitor sight; and the wearable equipment has the functions of heating massaging, light intensity monitoring and sitting posture correcting. The wearable equipment disclosed by the invention has the characteristics of being low in power consumption and portable.

However, the current lifestyle, digitalization and pandemic confinements limit an effective application of such a mechanism in a long-term perspective as a stand-alone solution. Consequently, alternative feature-specific treatments have been proposed for controlling the progression of myopia by modifying at least one specific feature that is, generally, assumed to contribute for this progression. Examples of such solutions include:
∘ an optical treatment, particularly by using a spectacle lens or a contact lens configured to manipulate at least one of a peripheral contrast, color, contrast, or an accommodation defocus signal;
∘ a radiation treatment, particularly by using an element configured for emitting a specific wavelength spectrum compensating at least one spectral portion in an intensity which is, typically, missing indoors; and
∘ a pharmaceutical treatment, particularly by applying a dose of atropine configured to manipulate accommodation signals, or choroidal thickening signals.

WO 2016/162554 A1 discloses a head mounted display device comprising: a light emitting source, an optical waveguide) adapted to collect light emitted from the light emitting source and to guide the collected light to the eye of a wearer when the head mounted display device is being worn by the wearer, a controller device adapted to control the emitted spectrum and/or radiance and/or light level emitted by the light emitting source.

WO 2021/163343 A2 discloses a method, a system and a computer-readable medium of utilizing artificial lighting regulating a circadian clock of a human. By utilizing light emitting diodes (LEDs) that emit wavelengths associated with human opsin absorption spectra, a device may stimulate opsins in the human. For example, the device may replicate normal sunlight (e.g., based on season or time of day) by emitting light that changes in rhythmic intensity or spectral modulation.

WO 2022/018080 A1 discloses a monitoring equipment comprising an eyewear that includes: a frame; at least one lens that is fixed to the frame and that is able to change the natural evolution of an optical deficiency; a wearing sensor able to determine if the eyewear is being worn by a wearer; and a processing unit that is programmed to: acquire the data determined by the wearing sensor; deduce therefrom a parameter relative to the length of time the wearer has worn the eyewear during a predetermined period; and compare said parameter with at least a predetermined datum to determine a level of efficiency of the optical deficiency treatment.

However, Varnas S, Gu X, Metcalfe A., Bayesian Meta-Analysis of Myopia Control with Multifocal Lenses, J Clin Med. 2021; 10(4):730 have shown that the known feature-specific treatments are limited by being only partially effective when deployed as a stand-alone treatment, particularly since their efficacies tend to decrease over time.

The above-mentioned treatments of myopia provide a temporally static alteration of a particular feature. For the first few months or years of the treatment, sensory units of the person under treatment which are sensitive to altered features, typically, respond well but eventually adapt to it. In a disadvantage manner, this leads to a decreasing effect of the efficacies. Additionally, once a particular treatment is withdrawn, the altered features for the treatment period goes back to their natural state. As the natural state has been less probable during the treatment period, the sensory units respond even higher during withdrawal, thus leading to rebounded myopia progression effect.

Further, Tong L. et al., Atropine for the Treatment of Childhood Myopia: Effect on Myopia Progression after Cessation of Atropine, Ophthalmology 2009; 116:572-579; Cho P, Cheung SW, Discontinuation of orthokeratology on eyeball elongation (DOEE), Cont Lens Anterior Eye. 2017; 40:82-87; and Lam, Carly SY, et al., Myopia control in children wearing DIMS spectacle lens: 6 years results, Investigative Ophthalmology & Visual Science 2022; 63.7:4247-4247 have demonstrated that a rebound effect usually occurs after withdrawal of a particular treatment.

### Problem to be solved

It is therefore an objective of the present invention to provide a system, a method, and a computer program for controlling a progression of myopia in at least one eye of a person, which at least partially overcome the limitations of the state of the art.

It is a particular objective of the present invention to provide a system, a method, and a computer program which are capable of tackling myopia progression in a sustainable and efficient manner by taking into account the underlying factors of the decrease in efficacy overtime and by avoiding or reducing the rebound effect.

### Summary of the invention

This problem is solved by a system, a method, and a computer program for controlling a progression of myopia in at least one eye of a person having the features of the independent claims. Preferred embodiments, which can be implemented in an isolated fashion or in any arbitrary combination, are listed in the dependent claims and throughout the following description.

In a first aspect, a method for controlling a progression of myopia in at least one eye of a person is disclosed. According to the present invention, the method is comprising the following steps:
- applying a treatment process during a period of time, wherein the treatment process is designed for reducing the progression of myopia in at least one eye of a person, wherein the treatment process is determined by at least one characteristic parameter, wherein the treatment process is selected from a class of treatment processes comprising:
   ∘ an optical treatment of the at least one eye of the person;
   ∘ a radiation treatment of the at least one eye of the person;
   ∘ a pharmaceutical treatment of the at least one eye of the person; and
   ∘ a behavioral treatment of the person,
   and
- determining at least one result of the treatment process on the progression of the myopia in the at least one eye of the person,
wherein at least two treatment processes, each selected from a different class, are simultaneously applied to the person during a first period of time.

As generally used, the term "refractive value" corresponds to at least one refractive error of at least one eye of a person, which can, particularly, be used for producing at least one spectacle lens that exhibits, based on the refractive values, a refractive power which is capable of correcting the at least one refractive error of the at least one eye of the person. Instead of the term "person", a different term, such as ʺʺuser", "subject", "individual", or "wearer", may also be applicable. As used herein, the term "at least one eye" refers to one eye or to both eyes of the person. Based on standard ISO 13666:2019, referred herein as the "Standard", Section 3.5.2, the term "spectacle lens" refers to an optical lens which is used for correcting the at least one refractive error of the at least one eye of the person, wherein the optical lens is carried in front of the eye of the person, thereby avoiding a direct contact with the eye of the person. According to the present invention, the refractive error of the at least one eye of the person has a value related to a spherical power and may, in addition, have at least one further value related to at least one of:
- a cylindrical power;
- a cylinder axis; or
- an addition power.

Based on the Standard, Section 3.12.2, the term "spherical power", usually abbreviated to "sphere" or "sph", refers to a value of a back vertex power of a spherical-power lens, or for a back vertex power in one of two principal meridians of an astigmatic-power lens, depending on a principal meridian chosen for reference. The spherical power of the at least one eye of the person may be a value related to a "spherical equivalent". As based on the Standard, Section 3.13.7, the term "cylindrical power", usually abbreviated to "cylinder" or "cyl", refers to an algebraic difference between principal powers with power of the principal meridian chosen for reference being subtracted from the other principal power. As based on in the Standard, Section 3.13.8, the term "cylinder axis", usually abbreviated to "cyl axis" or "axis", refers to a direction of the principal meridian of a lens whose vertex power is chosen for reference. As based on in the Standard, Section 3.16.3, the term "addition power", "addition" also abbreviated to "add", refers to a difference between the vertex power of a near portion and the vertex power of a distance portion in a multifocal or power-variation lens. The addition power may further refer to a difference between the vertex power of an intermediate portion and the vertex power of a distance portion in a multifocal or power-variation lens. The addition power may further refer to a single vision lens having a vertex power that is defined for correcting vision at a distance for near vision or intermediate vision.

Further, the term "spectacle frame" refers to a mount which is designed for fixedly receiving a pair of the spectacle lenses. For this purpose, the spectacle frame can have a receptacle on a right side and on a left side for each spectacle lens. The spectacle frame may comprise a transparent or non-transparent material that may, preferably, be solid but flexible and light. In general, a distinction can be made between "full-rim glasses" in which the frame encloses the pair of the spectacle lenses, "half-rim glasses" in which the pair of the spectacle lenses is only partially attached to the frame, and "frameless spectacles" in which each spectacle lens is only attached to the spectacle frame by using a support. By removably attaching the spectacle frame to the head of the wearer, particularly at spatial locations at the nose and at each ear of the wearer, at least three individual placement areas may be defined. As used herein, the term "placement area" refers to a spatial location at which the spectacle frame touches a portion of the head of the wearer, in particular at the spatial locations at the nose and at each ear of the wearer, in order to removably attach the spectacle frame to the head of the wearer. In addition, the spectacle frame may comprise further components, in particular a pair of temples for wearing the eyeglasses on the ears and a pair of nose pads for wearing the eyeglasses on the nose of the person. Herein, each nose pad can be formed as part of the spectacle frame or, as an alternative, provided via a separate mount, typically, denoted as "pad arm". However, for the purposes of the present invention the further components of the spectacle frame may, preferably, remain outside consideration.

Disclosed herein is a method for controlling a progression of myopia in at least one eye of a person. As generally used, the term "myopia" relates to a refractive status of a "person in which a refractive power of at least one eye of the person assumes a value below -0.5 dpt.

As further generally used, each of the terms "progression of myopia" or "myopia progression" refers to a temporal alteration, in particular a decrease, especially a monotonous decrease, of the refractive power of the at least one eye of the person over a period of time. Herein, the period of time may cover a number of years, preferred 1 year to 12 years, more preferred 2 years to 10 years, in particular 4 years to 8 years. However using a different period of time may also be feasible.

As further generally used, the term "controlling" or any grammatical variation thereof refers to a process combining a monitoring of a course of a variable and an influence the course of the variable by applying a treatment process during a period of time. As used herein, the term "treatment process" refers to a process of influencing the progression of myopia in at least one eye of a person, in particular at least one kind of preventive intervention for the at least one eye of the person, which is configured to at least one of decreasing myopia progression or retarding myopia onset in the at least one eye of the person. A particular treatment process is distinguished from a different treatment process using by at least one characteristic parameter describing at least one feature of the treatment process. As generally used, the term "characteristic parameter" refers to a value, in particular a numerical or an alphanumerical value, or a range of values which is configured to determine the particular treatment process. For examples of characteristic parameters, reference can be made to the description below.

The method for controlling a progression of myopia in at least one eye of a person comprises simultaneously applying at least two, in particular at least three, especially four, different treatment processes, each selected from a different class, to the person. As generally used, the term "simultaneous" or any grammatical variation thereof refers to applying at least two different processes during the same time interval. By way of example, the at least two different processes may be commenced and terminated at the point of time. As a further example, at least two of the different processes may be commenced and terminated at individual points of time, however, in a manner that a temporal overlap of applying the at least two different processes may occur.

As used herein, the term "class" refers to a category of treatment processes, wherein the treatment processes from the same class are based on the same technical principles by using the same or similar technical means configured for applying a particular treatment process. Further, a particular treatment process assigned to a particular class is distinguished from a further treatment process attributed to a different class by being based on a different technical principle by using other technical means configured for applying the particular treatment process. As disclosed herein, each treatment process applied to a person is selected from a class of treatment processes comprising:
∘ an optical treatment of the at least one eye of the person;
∘ a radiation treatment of the at least one eye of the person;
∘ a pharmaceutical treatment of the at least one eye of the person; and
∘ a behavioral treatment of the person.

As used herein, the term "optical treatment" refers to an application of at least one spherical lens to the at least one eye of the person. Preferably, the optical treatment may be selected from applying at least one of
- a peripheral contrast,
- a color,
- a contrast reduction, or
- an accommodation defocus signal
via at least one spectacle lens to the at least one eye of the person. For this purpose, the least one characteristic parameter of the optical treatment may, preferably, be selected from a parameter related to
- the peripheral contrast,
- the color,
- the contrast reduction, or
- the accommodation defocus signal
provided via the at least one spectacle lens to the at least one eye of the person. By way of example, the characteristic parameter of the optical treatment may be related to a particular color by indicating a transmission ratio over a range of wavelengths if incident light passing through the at least one spectacle lens in a direction towards the at least one eye of the person. Further examples are described below in more detail; however, still further examples are conceivable.

As described below in more detail, the optical treatment is, particularly, applied to the at least one eye of the person by using at least one spectacle lens configured for applying the optical treatment to the at least one eye of the person. For this purpose, the at least one spectacle lens may be adapted to meet at least one requirement imposed by the at least one characteristic parameter of the optical treatment. As further described below in more detail, the at least one spectacle lens that may be applied for the optical treatment of the at least one eye of the person may comprise at least two individual zones, wherein the at least one characteristic parameter of the optical treatment may be selected individually for each of the at least two zones in the at least one spectacle lens.

As further used herein, the term "radiation treatment" refers to an application of optical radiation to the at least one eye of the person. As generally used, the term "optical radiation" refers to electromagnetic waves having a wavelength of 380 nm to 780 nm, defining the so-denoted "optical wavelength range", wherein radiation from adjacent wavelength ranges, especially from 100 nm to below 380 nm, denoted as "long ultra-violet wavelength range", and from above 780 nm to 1.5 µm, denoted as "near infrared wavelength range" are included for the purposes of the present invention.

In particular, the radiation treatment may, preferably, comprise irradiating the at least one eye of the person by using light of a selected wavelength range. Herein, the least one characteristic parameter of the radiation treatment may, preferably, be selected from a parameter related to the wavelength range. In particular, the radiation treatment may be configured for providing a specific wavelength spectrum to the at least one eye of the person designed for compensating at least one spectral portion in an intensity which is, typically, missing indoors. By way of example, the characteristic parameter of the radiation treatment may indicate a particular wavelength range, such as 300 nm to 600 nm, or 400 nm to 600 nm, which may be used for irradiating the at least one eye of the person. However, selecting different values for the wavelength range may also be feasible. As described below in more detail, the radiation treatment may, particularly, be applied to the at least one eye of the person by using at least one light source, preferably at least one light-emitting diode, which may, preferably, be comprised by or attached to the spectacle frame.

As further used herein, the term "pharmaceutical treatment" refers to an application of a dose of at least one ophthalmic medication to the at least one eye of the person. Herein, the least one characteristic parameter of the pharmaceutical treatment may, preferably, comprise a value related to the dose of at least one ophthalmic medication to the at least one eye of the person. In particular, the at least one ophthalmic medication may be selected from atropine or cyclopentolate; however, using a different type of ophthalmic medication may also be feasible. As described below in more detail, the pharmaceutical treatment may, particularly, be applied to the at least one eye of the person by using at least one dispenser configured for applying a dose of at least one ophthalmic medication to the at least one eye of the person, wherein the at least one dispenser may, preferably, be comprised by or attached to the spectacle frame.

As further used herein, the term "behavioral treatment" refers to an application of at least one recommendation to the person. As generally used, the term "behavior" refers to a repeated activity of the person during which the at least one eye of the person is subject to an exposure of optical radiation. For a definition of the term "optical radiation", reference can be made to the definition as provided above. Not wishing to be bound by theory, Morgan I.G., et al., see above, indicate that evidence and causal relationship between the behavior of the person, on one hand, and myopia progression on the other hand, is strong and causal, especially depending on at least one parameter of the optical radiation, in particular related to an intensity, a spectral distribution and a duration of the optical radiation, incident on the at least one eye of the person. With particular regard to the present invention, the behavior of the person may, preferably, be selected from an amount of time spent by the person on at least one of
- near-vision working;
- outdoor activities or, vice-versa, on indoor activities.

However, using a different kind of behavior of the person may also be feasible.

As generally used, the term "near vision working" refers to a first type of repeated activity of the person dedicated to directing the eyes of the person to an object which is placed in a distance to the eyes of the person in a manner that the eyes of the person accommodate to a near point. As generally used, the term "accommodating" or any grammatical variation thereof relates to adjusting the refraction of the eyes of the person to a retinal plane of the eyes of the person when imaging an object located in front of the eyes of the person between a near point and a far point. The term "far point" relates to an end point of a refractive direction of the eyes of the person without accommodation, while the term "near point" refers to a point indicating a smallest distance in front of the eyes of the person at which the object can still be sharply imaged on the retinal plane of the eyes of the person, the near point being an individual quantity particularly depending on the age of the person. Herein, a fixed location of the eyes of the person, especially on the cornea, e.g. a location of an observable corneal reflex, can serve as reference point for measuring the distance of the near point to the eyes of the person. In practice, the object of the near vision working of the person may, in particular, be a literary object or a mobile communication device. As used herein, the term "literary object" refers to a kind of object comprising printed information, especially selected from a book, a brochure or a newspaper. Further, the term "mobile communication device" refers to at least one electronic device which is configured to present information by using an electronically driven screen, which can be carried by the person and, can thus, move together with the person, especially selected from a smartphone, a notebook, a personal digital assistant, or a laptop. However, further kinds of objects may also be feasible.

As further generally used, the term "outdoor activities" refers to a further type of repeated activity of the person, which is performed by the person outside a building in open air, especially during or after pre-school or school, on weekends, or during vacations. As indicated above, the intensity of the optical radiation which is incident on the at least one eye of the person can be considerably increased during an amount of time spent on outdoor activities by the person. Not wishing to be bound by theory, Morgan I.G., et al., see above, indicate that associations between the time spent on outdoor activities and a decrease of myopia progression are strong and consistently observed, while a controversy exists over whether increased time spent on outdoor activities not only reduces the myopia progression but also an onset of myopia. As further used herein, the term "indoor activities" refers to a further type of repeated activity of the person, which is performed by the person inside a building or a closed transport vehicle, such as train, a plane, or a car.

Preferably, the behavioral treatment of the person may be initiated by providing at least one biofeedback signal to the person, wherein the at least one biofeedback signal indicates a recommendation to the person, wherein the recommendation is intended to alter a behavior of the person. Herein, the least one characteristic parameter of the behavioral treatment of the person may preferably, be determined by using at least one value indicating the amount of time spent by the person on at least one of
- near-vision working; or
- outdoor activities or, vice-versa, on indoor activities.

A used herein, the term "amount of time" refers to a duration during which the respective repeated activity of the person is performed, especially by using average occupation of the person indicated in hours per day or hours per week. For a purpose of determining the amount of time, the period of time which is regularly spent outside vacations may be used; however it may be feasible to modify this value by adding the period of time, preferably in addition, spent outside during the vacations, thereby taking into account the annual duration of the vacations compared to the annual duration of the school time or the pre-school time.

By way of example, the least one characteristic parameter of the behavioral treatment of the person may be a threshold corresponding to a minimum amount of time spent on outdoor activities by the person within a particular time interval, such as a day or a week. In an event in which the amount of time spent on outdoor activities by the person may fall below the threshold within the particular time interval, a selected biofeedback signal may be provided to the person, thereby initiating the desired behavioral treatment of the person. As described below in more detail, at least one biofeedback device, in particular selected from a beeping element or a buzzing element, which is configured for generating the at least one biofeedback signal and for providing the at least one biofeedback signal to an attention of the person may be used, wherein the at least one biofeedback device may, preferably, be comprised by or attached to the spectacle frame. Similarly, the least one characteristic parameter of the behavioral treatment of the person may be a threshold corresponding to a maximum amount of time spent on near-vision working by the person within a particular time interval, such as a day or a week. In an event in which the amount of time spent on near-vision working by the person may exceed the threshold within the particular time interval, a selected biofeedback signal may be provided to the person, thereby initiating the desired behavioral treatment of the person. However, further examples are conceivable.

As already indicated above, at least two treatment processes, each selected from a different class, are simultaneously applied to the person during a first period of time, wherein each of the at least two treatment processes is determined by a value of the least one characteristic parameter. Herein, the same particular treatment process which is determined by a particular value of the least one characteristic parameter can be applied simultaneously applied to both eyes of the person. Alternatively or in addition, a further particular treatment process which is determined by a further particular value of the least one characteristic parameter can be applied only to a single eye of the person. Further, at least two different kinds of treatments selected from different classes, in particular from the optical treatment, the radiation treatment, or the pharmaceutical treatment, can be simultaneously applied. Herein, applying the at least two different kinds of treatments, particularly selected from an optical treatment, a radiation treatment, or a pharmaceutical treatment, can be simultaneously applied to a single eye or to both eyes of the person.

In a particularly preferred embodiment, at least two further treatment processes selected from the same two classes may, simultaneously, be applied to the person are during at least one subsequent period of time, wherein at least one of the at least two further treatment processes may differ from at least one preceding treatment process by the value of the least one characteristic parameter by which the at least one further treatment process may be determined. As generally used, the term "period of time" refers to a duration during which the at least two treatment processes or the at least two further treatment processes are concurrently applied. Herein, the period of time may be selected from a time of 1 month to 24 months, preferably of 3 months to 18 months, more preferred of 6 months to 15 months, particularly of 12 months. Herein, the period of time and at least one of the at least one subsequent period of time may differ from each other or may, preferably, be identical.

Herein, the value of at least one particular characteristic parameter by which a particular treatment process may be determined can, depending on the desired treatment of the person, differ from or be identical with the value of the least one particular characteristic parameter by which a preceding treatment process selected from the same class is determined. In other words, at least one of the at least two treatment processes can be maintained after the period of time has expired, while at least one further of the at least two treatment processes can be altered after the period of time. Respective example are described below in more detail.

Alternatively or in addition, two particular characteristic parameters determining a treatment process for each of the two eyes of the person, can be interchanged after the period of time. Herein, the value of at least one particular characteristic parameter by which a particular treatment process to a particular eye of the person is determined can be identical with the value of the least one particular characteristic parameter by which a preceding treatment process to the other of the particular eye of the person selected from the same class is determined. In this manner, a particular treatment of both eyes of the person can be altered between the two eyes of the person. In this manner, the first period of time can be considered as a "treatment period", while the second period of time can be considered as a "withdrawal period". Respective example are described below in more detail.

The method for controlling a progression of myopia in at least one eye of a person further comprises determining at least one result of the treatment process on the progression of the myopia in the at least one eye of the person. As generally used, the term "determining" or any grammatical variation thereof refers to a process of generating representative results which are, typically, denoted as "piece of data". As further generally used, the term of the at least one "result" refers to an outcome of the at least two treatment processes after the at least one amount of time. As described below in more detail, the piece of data, which correspond to the at least one result, can be based on measured information, which may, in particular, be recorded by using at least one detector. Herein, the at least one detector may, especially, be selected from an illumination sensor, a distance sensor configured for recording at least one distance of the at least one eye of the person in viewing direction to the at one spectacle lens, or an eye tracker configured for recording at least one movement of the at least one eye of the person. However, using a different type of detector may also be feasible.

In particular, the at least one result of at least two treatment processes in at least one preceding period of time can be considered as a piece of data which can, subsequently, be used for specifying the value of at least one value of the least one characteristic parameter in the at least one subsequent period of time. For this purpose, the piece of data can be evaluated from the at least one result of at least two treatment processes in the at least one preceding period of time.

The method as disclosed herein can, preferably, be a computer-implemented method. As generally used, the term "computer-implemented method" refers to a method involving at least one programmable device, particularly from a mobile communication device. However, a further kind of programmable device may also be feasible. Herein, the at least one programmable device may, in particular, comprise or have access to the processing device, wherein at least one of the features of the methods is performed by using at least one computer program. Preferably, the computer program may be provided on the at least one programmable device, or the at least one mobile communication device may have access to the computer program via a network, such as an in-house network or the internet.

In a further aspect, a computer program for controlling a progression of myopia in at least one eye of a person is disclosed, wherein the computer program comprises instructions which, when the program is executed by a computer, cause the computer to carry out the following steps:
- applying a treatment process during a period of time, wherein the treatment process is designed for reducing the progression of myopia in at least one eye of a person, wherein the treatment process is determined by at least one characteristic parameter, wherein the treatment process is selected from a class of treatment processes comprising:
   ∘ an optical treatment of the at least one eye of the person;
   ∘ a radiation treatment of the at least one eye of the person;
   ∘ a pharmaceutical treatment of the at least one eye of the person; and
   ∘ a behavioral treatment of the person,
   and
- determining at least one result of the treatment process on the progression of the myopia in the at least one eye of the person,
wherein at least two treatment processes, each selected from a different class, are simultaneously applied to the person during a first period of time.

Specifically, the computer program may be stored on a computer-readable, non-transitory data carrier, whereby any one of the method steps as indicated above may be performed by using a computer or a computer network, preferably by using a computer program.

In a further aspect, a computer program product is disclosed, computer program product has program code means stored on a machine-readable carrier, in order to perform the methods according to one or more of the embodiments disclosed herein, when the program is executed on a computer or computer network. As used herein, the term "computer program product" refers to the program as a tradable product. The product may generally exist in an arbitrary format, such as in a paper format, or on a computer-readable data carrier. Specifically, the computer program product may be distributed over a data network, such as the internet.

**In** a further aspect, a data carrier is disclosed, wherein the data carrier has a data structure stored thereon, which, after loading into a computer or computer network, such as into a working memory or main memory of the computer or computer network, may execute any one of the methods according to one or more of the embodiments disclosed herein.

In a further aspect, a modulated data signal is disclosed, wherein the modulated data signal comprises instructions readable by a computer system or computer network, for performing any one of the methods according to one or more of the embodiments as disclosed herein.

In a further aspect, a system for controlling a progression of myopia in at least one eye of a person is disclosed, the system comprising:
- a spectacle frame comprising a pair of spectacle lenses and at least one of the spectacle lenses incorporated by the spectacle frame, wherein the spectacle frame or at least one of the spectacle lenses are configured for applying a treatment process during a period of time in at least one eye of a person, wherein the treatment process is designed for reducing the progression of myopia in at least one eye of a person, wherein the treatment process is determined by at least one characteristic parameter, wherein the treatment process is selected from a class of treatment processes comprising:
   ∘ an optical treatment of the at least one eye of the person;
   ∘ a radiation treatment of the at least one eye of the person;
   ∘ a pharmaceutical treatment of the at least one eye of the person; and
   ∘ a behavioral treatment of the person,
   and
- a processing device configured for determining at least one result of the treatment process on the progression of the myopia in the at least one eye of the person,
wherein the spectacle frame or at least one of the spectacle lenses are configured for applying at least two treatment processes, each selected from a different class, simultaneously to the person during a first period of time.

As generally used, the term "system" refers to a combination of at least two components each of which is configured to perform a particular task, wherein, however, the at least two components may cooperate and/or interact with each other in order to achieve the desired task. As indicated above, the system at least comprises
- a spectacle frame comprising a pair of spectacle lenses;
- at least one spectacle lens incorporated by the spectacle frame; and
- a processing device,
wherein the processing device is, particularly, configured for determining at least one characteristic parameter to be used for controlling the spectacle frame or at least one zone of a particular spectacle lens for applying a treatment process during a period of time.

In a particularly preferred embodiment, the spectacle frame or at least one of the spectacle lenses may, further, be configured for applying, during at least one subsequent period of time, at least two further treatment processes selected from the same two classes simultaneously to the person, wherein at least one of the further treatment processes may differ from at least one preceding treatment process by a value of the least one characteristic parameter by which the treatment process may be determined. In order to provide a different optical treatment of the at least one eye of the person after a particular period of time, at least one of the spectacle lenses may be replaced in a manual fashion by manually removing the at least one spectacle lens from the spectacle frame and by, subsequently, introducing a different spectacle lens according to the at least one characteristic parameter by which the treatment process is determined into the spectacle frame.

However, in order to further facilitate providing a different optical treatment of the at least one eye of the person after a particular period of time, at least one characteristic parameter of the optical treatment may, as indicated above and below in more detail, be adjusted by selecting a parameter related to
- the peripheral contrast,
- the color,
- the contrast reduction, or
- the accommodation defocus signal
of the at least one spectacle lens to be provide to the at least one eye of the person. For this purpose, the spectacle lens may, preferably, be or comprise an electro-optical lens. As generally used, the term "electro-optical lens" refers to a type of optical lens having a refractive index that depends on an application of an external electrical voltage. Applying an external electrical voltage of a particular sign may result in a concave lens or a convex lens, wherein the value of the refractive index may depend on an amplitude of the applied external electrical voltage. Alternatively or in addition, the electro-optical lens may be configured for irradiating the at least one eye of the person by light of a selected wavelength range. The electro-optical lens may comprise a transparent ferroelectric material. The lens may be addressable as a whole. Alternatively, at least two zones in the at least one electro-optical lens may, individually, be addressable. However, using a different type of spectacle lenses may also be feasible.

The spectacle frame may, preferably, comprise at least one light source, preferably at least one light-emitting diode, which may, preferably, be comprised by or attached to the spectacle frame. In this manner, the spectacle frame may be designed for applying the radiation treatment to the at least one eye of the person by being configured for irradiating the at least one eye of the person by light of a selected wavelength range.

Further, at least one illumination sensor may, preferably, be comprised by or attached to the spectacle frame. Herein, the at least one illumination sensor may be configured for determining a value of illumination at the location of the at least one illumination sensor, in particular a lack of exposure of the person to daylight. Especially since the illumination of the person, typically, differs between indoor locations and outdoor locations, the value of illumination can be used for determining an amount of time spent on outdoor activities or, vice-versa, on indoor activities by the person wearing the spectacle frame.

Further, the spectacle frame may, preferably, be designed for applying the pharmaceutical treatment to the at least one eye of the person, especially by applying a dose of at least one ophthalmic medication to the at least one eye of the person. For this purpose, at least one dispenser configured for applying the dose of at least one ophthalmic medication to the at least one eye of the person may, preferably, be comprised by or attached to the spectacle frame. Further, at least one receptacle configured for storing a portion of the ophthalmic medication may, additionally, be comprised by or attached to the spectacle frame.

Further, the spectacle frame and the processing device may, further, be configured for initiating the behavioral treatment to the person, especially by providing at least one biofeedback signal to the person, thereby indicating a recommendation to the person being intended to alter a behavior of the person. For this purpose, at least one biofeedback device, particularly be selected from a beeping element or a buzzing element, which is configured for generating the at least one biofeedback signal and for providing the at least one biofeedback signal to an attention of the person and which is comprised by or attached to the spectacle frame may, preferably, be used. Herein, the processing device may, further, be configured for generating the at least one biofeedback signal based on the at least one value of at the least one characteristic parameter of the behavioral treatment of the person determined by the processing device.

Further, the spectacle frame and the processing device are may, further, be configured for recording at least one distance of the at least one eye of the person in viewing direction to the at one spectacle lens. For this purpose, at least one distance sensor may, preferably, be comprised by or attached to the spectacle frame. Herein, the processing device may, further, be configured for using a value recorded by the at least one distance sensor in determining an amount of time spent on near-vision working by the person and a recommendation to the person following therefrom as described elsewhere herein in more detail.

Further, the spectacle frame may, preferably, comprise at least one eye tracker which may be configured for recording at least one movement of the at least one eye of the person. Herein, the processing device may, further, be configured for using a value recorded by the at least one eye tracker in determining the recommendation to the person as described below in more detail.

As already indicated above, the system comprises a processing device which is, particularly, configured for determining at least one characteristic parameter to be used for controlling the spectacle frame or at least one of the spectacle lenses for, appropriately, applying a treatment process during a period of time. As used herein, the term "processing" or any grammatical variation thereof refers to applying at least one algorithm to data in a fashion that the desired at least two treatment processes each selected from a different class, are simultaneously applied to the person during at least two consecutive periods of time by using a value of the least one characteristic parameter by which a particular treatment process is determined. Herein, the at least one algorithm may be configured to determine the value of the least one characteristic parameter from at least one of a known or estimated preset value or a result of a preceding treatment on the progression of the myopia in the at least one eye of the person.

As further generally used, the term "processing device" refers to an apparatus which is designated for executing the at least one algorithm as described above, wherein the processing of the at least one algorithm may be performed in at least one of a consecutive or a parallel manner. According to a first embodiment, all method steps can be performed by using a single processing device, such as a computer, especially a stand-alone computer, or an electronic communication unit, specifically a smartphone, a tablet, a personal digital assistant, or a laptop. In this embodiment, the single processing device may be configured to exclusively perform at least one computer program, in particular at least one line of computer program code configured to execute at least one algorithm, as used in at least one of the methods according to the present invention. Herein, the computer program as executed on the single processing device may comprise all instructions causing the computer to carry out the at least one of the methods according to the present invention. Alternatively or in addition, at least one method step can be performed by using at least one remote processing device, especially selected from at least one of a server or a cloud computer, which is not located at the site of the person when executing the at least one method step. In further embodiment, the computer program may comprise at least one remote portion to be executed by the at least one remote processing device to carry out the at least one method step. Further, the computer program may comprise at least one interface configured to forward and/or receive data to and/or from the at least one remote portion of the computer program.

Further, the processing device may, preferably, comprise at least one communication interface configured to provide communication with at least one of the spectacle frame, apportion thereof, or a user, wherein the user may, preferably, be selected from at least one of an eye care professional, specifically an optician, optometrist or ophthalmologist, the person subject to myopia, or a related person, especially a parent of the person or a nurse caring for the person. As generally used, the term "communication interface" refers a transmission channel being designated for a transmission of data. Preferably, the communication interface may be arranged as a unidirectional interface which is configured to forward at least one piece of data into a single direction, from the at least one input interface to the processing device, or from the processing device to the at least one output interface. Alternatively, the communication interface may be arranged as a bidirectional interface which is configured to forward at least one piece of data into one of two directions, from a communication unit, which may comprise both the input interface and the output interface, to the processing device, or vice versa.

For a purpose of data transmission, the communication interface may comprise at least one of an input interface or an output interface. As generally used, the term "input interface" refers to an apparatus which is configured to receive at least one piece of data, specifically the data related to the treatment as described above or below in more detail. For this purpose, the data can, preferably, be provided in form of at least one of an input file or as input data by using a graphical user interface (GUI), and forwarded to the processing device for determining the desired data related to the progression of the refractive values of the person. As generally used, the terms "graphical user interface" or "GUI" refer to a type of input interface which is configured to receive the desired data from a graphical interaction with a user. The graphical interaction may comprise presenting graphical icons on a screen to the user, recording a reaction of the user, and determining the desired input data by evaluating the reaction of the user. For this purpose, at least one touchscreen configured to provide access to inputting at least one piece of data, specifically the data related to the person, may be used. However, other devices may also be feasible.

As further generally used, the term "output interface" refers to a further apparatus which is configured to provide at least one output file comprising at least one further piece of data, specifically the desired data related to the at least one result of the treatment on the progression of the myopia in the at least one eye of the person. Herein, the processing device may, preferably, be configured to provide the data related to the progression of the refractive values of the person via the by using, preferably, the same or a different graphical user interface which displays the data comprised by the output file to the user, in particular by using a graphical user interface, preferably the same graphical user interface as used for the input interface. However, a further kind of output interface may also be feasible.

In a particular embodiment, the spectacle frame may comprise the processing device completely or a portion thereof. In this embodiment, the spectacle frame or the portion thereof may, further, comprise at least one communication interface configured for at least one of forwarding or receiving data.

In a further aspect, a method for producing at least one spectacle lens is disclosed. For this purpose, at least one lens blank is processed by using at least one manufacturing device which employs data related to refractive values as determined from the at least one result of the treatment on the progression of the myopia in the at least one eye of the person as determined by using the method , in particular, by using the processing device, wherein the data are forwarded to the at least one manufacturing device as well-known by the person skilled in the art.

For further details with respect to the system and the computer-related aspects for controlling a progression of myopia in at least one eye of a person, reference can be made to the description of the method for controlling the progression of myopia in at least one eye of a person throughout this document.

With respect to the prior art, the system, the method, and the computer program for controlling the progression of myopia in at least one eye of a person according to the present invention exhibit various advantages. In particular, they are capable of tackling the progression of myopia in a sustainable and efficient manner, whereby the underlying factors of the decrease in efficacy overtime and by avoiding or reducing the rebound effect are taken into account. In particular, the system, the method, and the computer program according to the present invention allows different types of treatment of one eye or both eyes of the person in a simultaneous manner, which can be altered after a period of time. As a result thereof, the system, the method, and the computer program as disclosed herein are, advantageously, adapted for concurrently
- sustaining and boosting efficacies of myopia solutions by mitigating adaptation; and
- preventing a rebound effect.

As used herein, the terms "have", "comprise" or "include" or any arbitrary grammatical variations thereof are used in a non-exclusive way. Thus, these terms may refer to both a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

As further used herein, the terms "preferably", "more preferably", "particularly", "more particularly", or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in this way with other features of the invention.

Summarizing, the following Embodiments are particularly preferred within the scope of the present invention:
Embodiment 1: A method for controlling a progression of myopia in at least one eye of a person, the method comprising the following steps:
   - applying a treatment process during a period of time, wherein the treatment process is designed for reducing the progression of myopia in at least one eye of a person, wherein the treatment process is determined by at least one characteristic parameter, wherein the treatment process is selected from a class of treatment processes comprising:
      ∘ an optical treatment of the at least one eye of the person;
      ∘ a radiation treatment of the at least one eye of the person;
      ∘ a pharmaceutical treatment of the at least one eye of the person; and
      ∘ a behavioral treatment of the person,
      and
   - determining at least one result of the treatment process on the progression of the myopia in the at least one eye of the person,
   wherein at least two treatment processes, each selected from a different class, are simultaneously applied to the person during a first period of time.
Embodiment 2: The method according to the preceding Embodiment, wherein, during at least one subsequent period of time, at least two further treatment processes selected from the same two classes are simultaneously applied to the person, wherein at least one of the further treatment processes differs from at least one preceding treatment process by a value of the least one characteristic parameter by which the treatment process is determined.
Embodiment 3: The method according to any one of the preceding Embodiments, wherein, the first period of time is a treatment period, and wherein the second period of time is a withdrawal period.
Embodiment 4: A computer program for controlling a progression of myopia in at least one eye of a person, wherein the computer program comprises instructions which, when the program is executed by a computer, cause the computer to carry out the following steps:
   - applying a treatment process during a period of time, wherein the treatment process is designed for reducing the progression of myopia in at least one eye of a person, wherein the treatment process is determined by at least one characteristic parameter, wherein the treatment process is selected from a class of treatment processes comprising:
      ∘ an optical treatment of the at least one eye of the person;
      ∘ a radiation treatment of the at least one eye of the person;
      ∘ a pharmaceutical treatment of the at least one eye of the person; and
      ∘ a behavioral treatment of the person,
      and
   - determining at least one result of the treatment process on the progression of the myopia in the at least one eye of the person,
   wherein at least two treatment processes, each selected from a different class, are simultaneously applied to the person during a first period of time.
Embodiment 5: The computer program according to the preceding Embodiment, wherein, during at least one subsequent period of time, at least two further treatment processes selected from the same two classes are simultaneously applied to the person, wherein at least one of the further treatment processes differs from at least one preceding treatment process by a value of the least one characteristic parameter by which the treatment process is determined.
Embodiment 6: The method according to any one of the preceding Embodiments, wherein, the first period of time is a treatment period, and wherein the second period of time is a withdrawal period.
Embodiment 7: The computer program according to any one of the preceding Embodiments, wherein at least three treatment processes, each selected from a different class, are simultaneously applied to the person.
Embodiment 8: The computer program according to any one of the preceding Embodiments, wherein four treatment processes, each selected from a different class, are simultaneously applied to the person.
Embodiment 9: The computer program according to any one of the preceding Embodiments, wherein the value of at least one particular characteristic parameter by which a particular treatment process is determined differs from or is identical with the value of the least one particular characteristic parameter by which a preceding treatment process selected from the same class is determined.
Embodiment 10: The computer program according to any one of the preceding Embodiments, wherein at least two of the optical treatment, the radiation treatment, or the pharmaceutical treatment are provided simultaneously to both eyes of the person.
Embodiment 11: The computer program according to the preceding Embodiment, wherein the value of at least one particular characteristic parameter by which a particular treatment process to a particular eye of the person is determined is identical with the value of the least one particular characteristic parameter by which a preceding treatment process to the other of the particular eye of the person selected from the same class is determined.
Embodiment 12: The computer program according to any one of the preceding Embodiments, wherein the optical treatment is selected from applying at least one of:
   - a peripheral contrast;
   - a color;
   - a contrast reduction; or
   - an accommodation defocus signal
   via at least one spectacle lens to the at least one eye of the person.
Embodiment 13: The computer program according to the preceding Embodiment, wherein the least one characteristic parameter of the optical treatment is selected from a parameter related to:
   - the peripheral contrast;
   - the color;
   - the contrast; or
   - the accommodation defocus signal
   provided via the at least one spectacle lens to the at least one eye of the person.
Embodiment 14: The computer program according to any one of the preceding Embodiments, wherein the at least one characteristic parameter of the optical treatment is selected individually for at least one zone in the at least one spectacle lens applied for the optical treatment of the at least one eye of the person.
Embodiment 15: The computer program according to any one of the preceding Embodiments, wherein the radiation treatment comprises irradiating the at least one eye of the person by using light of a selected wavelength range.
Embodiment 16: The computer program according to the preceding Embodiment, wherein the least one characteristic parameter of the radiation treatment is selected from a parameter related to the wavelength range.
Embodiment 17: The computer program according to any one of the preceding Embodiments, wherein the pharmaceutical treatment comprises applying a dose of at least one ophthalmic medication to the at least one eye of the person.
Embodiment 18: The computer program according to the preceding Embodiment, wherein the least one characteristic parameter of the pharmaceutical treatment comprises a value related to the dose of at least one ophthalmic medication to the at least one eye of the person.
Embodiment 19: The computer program according to any one of the two preceding Embodiments, wherein the ophthalmic medication is atropine or cyclopentolate.
Embodiment 20: The computer program according to any one of the preceding Embodiments, wherein the behavioral treatment of the person is initiated by providing at least one biofeedback signal to the person, wherein the at least one biofeedback signal indicates a recommendation to the person, wherein the recommendation is intended to alter a behavior of the person.
Embodiment 21: The computer program according to the preceding Embodiment, wherein the behavior of the person refers to a repeated activity of the person during which the at least one eye of the person is subject to an exposure of optical radiation.
Embodiment 22: The computer program according to any one of the two preceding Embodiments, wherein the behavior of the person is selected from an amount of time spent by the person on at least one of
   - near-vision working; or
   - outdoor activities or, vice-versa, on indoor activities.
Embodiment 23: The computer program according to any one of the three preceding Embodiments, wherein the least one characteristic parameter of the behavioral treatment of the person is determined by using at least one value indicating the amount of time spent by the person on at least one of:
   - near-vision working; or
   - outdoor activities or, vice-versa, on indoor activities.
Embodiment 24: The computer program according to any one of the preceding Embodiments, wherein both the period of time and the at least one subsequent period of time are selected from a time of 1 month to 24 months, preferably of 3 months to 18 months, more preferred of 6 months to 15 months, particularly of 12 months.
Embodiment 25: The computer program according to any one of the preceding Embodiments, wherein the period of time and at least one of the at least one subsequent period of time are identical.
Embodiment 26: The computer program according to any one of the preceding Embodiments, further comprising a determining of the at least one value of the least one characteristic parameter in the at least one subsequent period of time by using the at least one result of the at least two treatment processes in at least one preceding period of time.
Embodiment 27: The computer program according to the preceding Embodiment, wherein the computer-program is stored on a non-transitory computer readable storage medium.
Embodiment 28: A system for controlling a progression of myopia in at least one eye of a person, the system comprising:
   - a spectacle frame comprising a pair of spectacle lenses and at least one of the spectacle lenses incorporated by the spectacle frame, wherein the spectacle frame or at least one of the spectacle lenses are configured for applying a treatment process during a period of time, wherein the treatment process is designed for reducing the progression of myopia in at least one eye of a person, wherein the treatment process is determined by at least one characteristic parameter, wherein the treatment process is selected from a class of treatment processes comprising:
      ∘ an optical treatment of the at least one eye of the person;
      ∘ a radiation treatment of the at least one eye of the person;
      ∘ a pharmaceutical treatment of the at least one eye of the person; and
      ∘ a behavioral treatment of the person,
      and
   - a processing device configured for determining at least one result of the treatment process on the progression of the myopia in the at least one eye of the person,
   wherein the spectacle frame or at least one of the spectacle lenses are configured for applying at least two treatment processes, each selected from a different class, simultaneously to the person during a first period of time.
Embodiment 29: The system according to the preceding Embodiment, wherein the spectacle frame comprises the processing device completely or a portion thereof.
Embodiment 30: The system according to the preceding Embodiment, wherein the spectacle frame or the portion thereof further comprises at least one communication interface configured for at least one of forwarding or receiving data.
Embodiment 31: The system according to any one of the preceding system Embodiments, wherein the spectacle frame or at least one of the spectacle lenses are further configured for applying, during at least one subsequent period of time, at least two further treatment processes selected from the same two classes simultaneously to the person, wherein at least one of the further treatment processes differs from at least one preceding treatment process by a value of the least one characteristic parameter by which the treatment process is determined.
Embodiment 32: The system according to any one of the preceding system Embodiments, wherein the spectacle frame or at least one of the spectacle lenses are configured for applying at least three treatment processes, each selected from a different class, simultaneously to the person.
Embodiment 33: The system according to any one of the preceding system Embodiments, wherein the spectacle frame or at least one of the spectacle lenses are configured for applying four treatment processes, each selected from a different class, simultaneously to the person.
Embodiment 34: The system according to any one of the preceding system Embodiments, wherein the spectacle frame or at least one of the spectacle lenses are configured for applying
   - at least one of the optical treatment, the radiation treatment, or the pharmaceutical treatment simultaneously to both eyes of the person;
   - at least one of a different optical treatment, a different radiation treatment, or a different pharmaceutical treatment to each eye of the person.
Embodiment 35: The system according to any one of the preceding system Embodiments, wherein the spectacle lenses are designed to provide an optical treatment by application of at least one of
   - a peripheral contrast;
   - a color;
   - a contrast reduction; or
   - an accommodation defocus signal
   to the at least one eye of the person.
Embodiment 36: The system according to any one of the preceding system Embodiments, wherein at least one of the spectacle lenses is or comprises an electro-optical lens.
Embodiment 37: The system according to the preceding Embodiment, wherein at least two zones in the at least one electro-optical lens are individually addressable.
Embodiment 38: The system according to any one of the preceding system Embodiments, wherein the spectacle frame is designed for applying the radiation treatment to the at least one eye of the person by being configured for irradiating the at least one eye of the person by light of a selected wavelength range.
Embodiment 39: The system according to the preceding Embodiment, wherein at least one of the spectacle lenses is or comprises an electro-optical lens configured for irradiating the at least one eye of the person by light of a selected wavelength range.
Embodiment 40: The system according to any one of the two preceding Embodiments, wherein the spectacle frame further comprises at least one light source, preferably at least one light-emitting diode.
Embodiment 41: The system according to any one of the three preceding Embodiments, wherein the spectacle frame further comprises at least one illumination sensor.
Embodiment 42: The system according to any one of the preceding system Embodiments, wherein the spectacle frame is further designed for applying the pharmaceutical treatment by applying a dose of at least one ophthalmic medication to the at least one eye of the person.
Embodiment 43: The system according to the preceding Embodiment, wherein the spectacle frame further comprises at least one dispenser configured for applying the dose of at least one ophthalmic medication to the at least one eye of the person.
Embodiment 44: The system according to any one of the two preceding Embodiments, wherein the spectacle frame further comprises at least one receptacle configured for storing a portion of the ophthalmic medication.
Embodiment 45: The system according to any one of the preceding system Embodiments, wherein the spectacle frame and the processing device are further configured for initiating the behavioral treatment to the person by providing at least one biofeedback signal to the person, wherein the at least one biofeedback signal indicates a recommendation to the person, wherein the recommendation is intended to alter a behavior of the person.
Embodiment 46: The system according to the preceding Embodiment, wherein the processing device is further configured for generating the at least one biofeedback signal based on the at least one value of at the least one characteristic parameter of the behavioral treatment of the person determined by the processing device.
Embodiment 47: The system according to the preceding Embodiment, wherein the at least one value the at least one value indicates an amount of time spent by the person on at least one of:
   - near-vision working; or
   - outdoor activities or, vice-versa, on indoor activities.
Embodiment 48: The system according to any one of the three preceding Embodiments, wherein the spectacle frame further comprises at least one biofeedback device, wherein the at least one biofeedback device is configured for
   - generating the at least one biofeedback signal; and
   - providing the at least one biofeedback signal to an attention of the person.
Embodiment 49: The system according to any one of the three preceding Embodiments, wherein the at least one biofeedback device is selected from at least one of:
   - a beeping element; or
   - a buzzing element,
   comprised by or attached to the spectacle frame.
Embodiment 50: The system according to any one of the preceding system Embodiments, further comprising at least one distance sensor configured for recording at least one distance of the at least one eye of the person in viewing direction to the at one spectacle lens.
Embodiment 51: The system according to the preceding Embodiment, wherein the processing device is further configured for using a value recorded by the at least one distance sensor in determining the recommendation to the person.
Embodiment 52: The system according to any one of the preceding system Embodiments, further comprising at least one eye tracker configured for recording at least one movement of the at least one eye of the person.
Embodiment 53: The system according to the preceding Embodiment, wherein the processing device is further configured for using a value recorded by the at least one eye tracker in determining the recommendation to the person.
Embodiment 54: The system according to any one of the preceding system Embodiments, wherein the processing device is further configured for:
   - selecting the period of time and the at least one subsequent period of time; and
   - determining the at least one value for the at least one characteristic parameter.
Embodiment 55: The system according to the preceding Embodiment, wherein the processing device is further configured for determining the at least one value of the least one characteristic parameter in the at least one subsequent period of time by using the at least one result of the at least two treatment processes in at least one preceding period of time.
Embodiment 56: The system according to any one of the preceding system Embodiments, wherein the processing device is further configured for:
   - providing communication between at least two parts of the system;
   - generating at least one signal for modulating the at least one electro-optical lens;
   - switching the at least one light source, preferably the at least one light-emitting diode;
   - controlling the at least one dispenser; and
   - recording at least one signal received from the at least one eye-tracker.

### Short description of the Figures

Further optional features and embodiments of the present invention are disclosed in more detail in the subsequent description of preferred embodiments, preferably in conjunction with the dependent claims. Therein, the respective optional features may be implemented in an isolated fashion as well as in any arbitrary feasible combination, as the skilled person will recognize. It is emphasized here that the scope of the invention is, however, not restricted to the preferred embodiments. In the Figures:
- Figure 1: illustrates an exemplary embodiment of a system for controlling a progression of myopia in at least one eye of a person according to the present invention;
- Figure 2: illustrates exemplary embodiments of a pair of spectacle lenses designed in accordance with the present invention;
- Figure 3: illustrates further exemplary embodiments of a spectacle lens designed in accordance with the present invention; and
- Figure 4: illustrates exemplary treatment schemes for controlling the progression of myopia in the at least one eye of the person according to the present invention.

### Detailed description of the embodiments

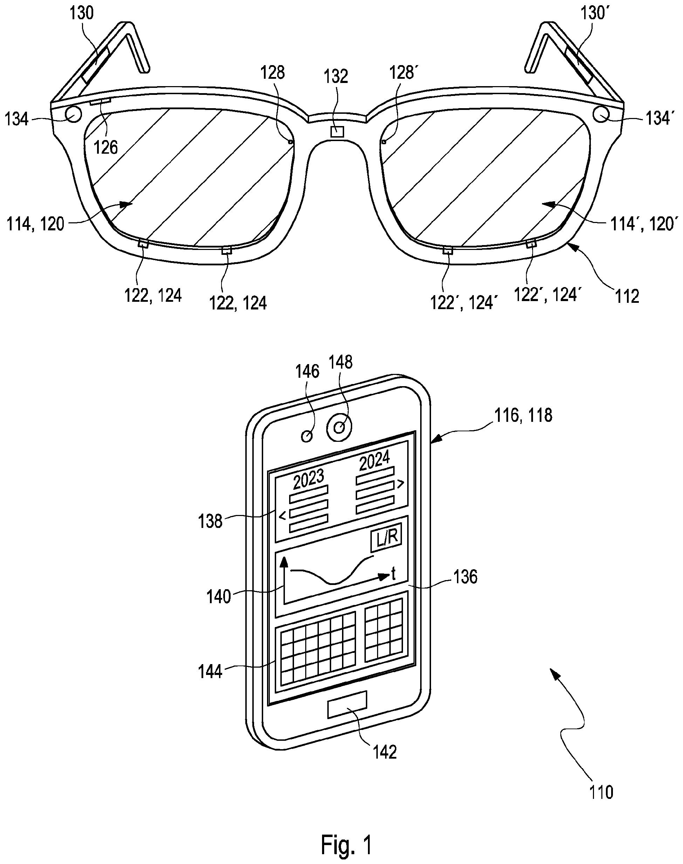

Figure 1 illustrates an exemplary embodiment of a system 110 for controlling a progression of myopia in at least one eye of a person according to the present invention. As schematically depicted there, the system comprises a spectacle frame 112, which comprises a pair of spectacle lenses 114, 114, and the pair of the spectacle lenses 114, 114' incorporated by the spectacle frame 112, wherein the spectacle frame 112 and both spectacle lenses 114, 114' are configured for applying treatment processes to a person during a period of time, wherein each treatment process is designed for reducing the progression of myopia, as well as a processing device 116, wherein the processing device 116 is configured for determining results of the treatment processes on the progression of the myopia in the at least one eye of the person. As depicted, the processing device 116 is comprised here by a smartphone 118; however using a different type of processing device 116 may also be feasible.

According to the present invention, two, three, or four treatment processes are simultaneously applied to the person during a period of time, wherein each treatment process is selected from a different class of treatment processes, i.e. from:
∘ an optical treatment of one eye or both eyes of the person;
∘ a radiation treatment of one eye or both eyes of the person;
∘ a pharmaceutical treatment of one eye or both eyes of the person; and
∘ a behavioral treatment of the person.

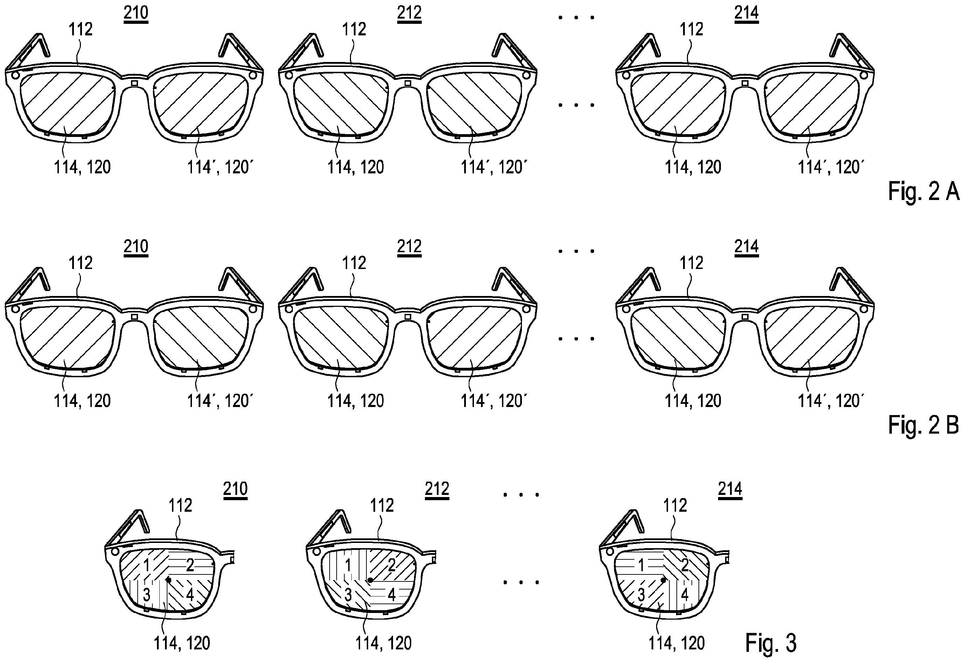

In order to apply an optical treatment to one eye or both eyes of the person, both spectacle lenses 114, 114' as illustrated in Figure 1 are or comprise electro-optical lenses 120, 120'. However, embodying only one spectacle lens as an electro-optical lens may also be feasible. Since the refractive index of an electro-optical lens 120, 120' depends on an application of an external electrical voltage, applying an external electrical voltage of a particular sign may result in a concave lens or a convex lens, wherein the value of the refractive index may depend on an amplitude of the applied external electrical voltage. As schematically illustrated in Figures 1, 2A and 2B, each electro-optical lens 120, 120' is addressable as a whole. Alternatively, two or more zones in one eye or both electro-optical lenses 120, 120', such as the four zones as depicted in Figure 3 in both electro-optical lenses 120, 120', are individually addressable. In this manner, the electro-optical lenses 120, 120' as depicted in Figures 1 to 3 are designed to be able to apply at least one particular peripheral contrast, color, contrast and/or an accommodation defocus signal to one eye or both eyes of the person. However, using a different type of spectacle lenses 114, 114' for this purpose may also be feasible.

In order to apply a radiation treatment to one eye or both eyes of the person, the spectacle frame 112 as illustrated in Figure 1 further comprises light sources 122, 122'. As depicted there, the light sources 122, 122' in this exemplary embodiment are light-emitting diodes 124, 124' comprised by or attached to the spectacle frame 112; however using a different kind of light sources 122, 122' may also be feasible. In this manner, the light sources 122, 122' are configured for irradiating one eye or both eyes of the person by using light of a selected wavelength range, in particular selected from optical radiation having a wavelength of 380 nm to 780 nm, wherein radiation from an adjacent wavelength range, especially from the long ultra-violet wavelength range of 100 nm to below 380 nm and/or from the near infrared wavelength range of above 780 nm to 1.5 µm, can be included for the purposes of the present invention.

Alternatively or in addition, at least one of the electro-optical lenses 120, 120' as described above may be configured for irradiating the one eye or both eye of the person by light of a selected wavelength range.

As further illustrated in Figure 1, the spectacle frame 112 further comprises an illumination sensor 126, which may, preferably, be comprised by or attached to the spectacle frame 112. The illumination sensor 126 may, preferably, be configured to track an exposure of the person to daylight, whereinafter the light sources 122, 122' may compensate the lack of exposure of the person to daylight by irradiating one eye or both eyes of the person by using light of a selected wavelength range. In this manner, the radiation treatment may be configured for providing a specific wavelength spectrum to the at least one eye of the person designed for compensating at least one spectral portion in an intensity which is, typically, missing indoors.

In order to apply a pharmaceutical treatment to one eye or both eyes of the person, the spectacle frame 112 as illustrated in Figure 1 further comprises dispensers 128, 128', which are configured for applying a dose of an ophthalmic medication to the one eye or to both eyes of the person, especially by spraying a determined dose to a particular eye of the person. As depicted there, the dispensers 128, 128' in this exemplary embodiment are comprised by or attached to the spectacle frame 112. In addition, the spectacle frame 112 may, further, comprise one or more receptacles (not depicted here) configured for storing a portion of the ophthalmic medication. For this purpose, the one or more receptacles may be a compartment attached to the spectacle frame 112 or, preferably, a cavity comprised by a tubal portion of the spectacle frame 112 adapted to receive the portion of the ophthalmic medication to be stored therein.

For controlling the application of the desired dose of the ophthalmic medication to the one eye or to both eyes of the person, the processing device 116 may be used. Herein, the processing device 116 as comprised by the smartphone 118 may be used. Alternatively or in addition, the processing device 116 may, further, comprise one or more microcontrollers (not depicted here), which may be attached to or integrated into the spectacle frame 112. Herein, the processing device 116 may be configured for applying the desired dose of the ophthalmic medication to the one eye or to both eyes of the person depending on a characteristic parameter as determined of the pharmaceutical treatment, which may comprise a value related to the dose of the ophthalmic medication to the particular eye of the person.

However, further elements configured for storing and applying a dose of an ophthalmic medication to the one eye or to both eyes of the person as well as a controlling thereof are conceivable.

In order to trigger an application of a behavioral treatment to the person who is wearing the spectacle frame 112 comprising the spectacle lenses 114, 114', the spectacle frame 112 as illustrated in Figure 1 further comprises biofeedback device 130, 130'. For this purpose, the biofeedback devices 130, 130' may comprise a beeping element or a buzzing element that may be comprised by or attached to the spectacle frame 112. Herein, the biofeedback devices 130, 130' are configured for generating one or more biofeedback signals and for providing the one or more biofeedback signals to an attention of the person. Further, the processing device 116 may, additionally, be configured for generating the one or more biofeedback signals based on one or more values of one or more characteristic parameters of the behavioral treatment of the person determined by the processing device 116.

In this manner, the spectacle frame 112 and the processing device 116 are configured for initiating the behavioral treatment of the person by indicating a recommendation to the person intended to alter a behavior of the person. Herein, the one or more characteristic parameters of the behavioral treatment of the person may preferably, be determined by using at least one value indicating the amount of time spent by the person on at least one of:
- near-vision working; or
- outdoor activities or, vice-versa, on indoor activities.

As further depicted in Figure 1, the spectacle frame 112, additionally, has a distance sensor 132, which may, preferably, be comprised by or attached to the spectacle frame 112. In particular, the distance sensor 132 may be placed at a location of symmetry of the spectacle frame 112, such as the location on the spectacle frame 112 between the receptacles for the two spectacle lenses 114, 114' as schematically indicated in Figure 1. However, placing the distance sensor 132 at a different location on the spectacle frame 112 may also be feasible. Further, the processing device 116 may, especially, be configured for using a value recorded by the distance sensor 132 in determining the amount of time spent on near-vision working by the person and a corresponding recommendation to the person following therefrom. In an event in which the amount of time spent on near-vision working by the person may exceed the threshold within the particular time interval, a selected biofeedback signal may be provided to the person, thereby initiating the desired behavioral treatment of the person.

As already described above, the spectacle frame 112, additionally, has the illumination sensor 126, which may, preferably, be comprised by or attached to the spectacle frame 112. Since the illumination sensor 126 is configured to track an exposure of the person to daylight, the processing device 116 may, further, be configured for using a value recorded by the illumination sensor 126 in determining the amount of time spent by the person on outdoor activities or, vice-versa, on indoor activities, and a corresponding recommendation to the person following therefrom. In an event in which the amount of time spent on outdoor activities by the person may fall below the threshold within the particular time interval, a selected biofeedback signal may be provided to the person, thereby initiating the desired behavioral treatment of the person.

As further illustrated in Figure 1, the spectacle frame 112 comprises eye trackers 134, 134' for each eye of the person. Herein, the eye trackers 134, 134' may, especially, be configured for recording at least one movement of the at least one eye of the person. Herein, the eye trackers 134, 134' may, preferably, be used for monitoring at least one pupil of the person, in particular for determining a gaze position of the at least one eye of the person on a surface of a corresponding spectacle lens 114, 114'. In this manner, the at last one zone in the corresponding spectacle lens 114, 114' can, easily, be aligned to a gaze position of the at least one eye of the person during the optical treatment. Further, the processing device 116 may be configured for using a value recorded by the at least one eye tracker in determining the recommendation to the person.

As further schematically illustrated in Figure 1, the smartphone 118 may have a screen 136 which may be used as output interface configured for displaying information to the user, preferably selected from an eye care professional, specifically an optician, optometrist or ophthalmologist, the person subject to myopia, and/or a related person, especially a parent of the person or a nurse caring for the person. As depicted there, the further information may, preferably, comprise a graphic presentation 138 of a treatment scheme and a diagram 140 showing the results of the treatment process on the progression of myopia in the at least one eye of the person. Further, a loudspeaker 142 may also be used as output interface. In addition, the screen 136 may, further, be used as input interface configured for displaying information to the user. For this purpose, the screen 136 may, as further depicted there, comprise a virtual keyboard 144. Further, a microphone 146 and/or a front camera 148 may also be used as input interface. However, further embodiments with respect to the input interface and the output interface may be conceivable.

Figure 2 illustrates exemplary embodiments of a pair of spectacle lenses 114, 114', which are designed in accordance with the present invention.

In the exemplary embodiment of Figure 2A, both spectacle lenses 114, 114' exhibit the same optical treatment for both eyes of the person during a first period of time 210. During a second period of time 212, both spectacle lenses 114, 114' still exhibit the same optical treatment for both eyes of the person, which, however, differs from the optical treatment for both eyes of the person during the first period of time 210. For this purpose, the refractive values of each spectacle lens 114, 114' can be altered, preferably by switching the electro-optical lenses 120, 120'. While the first period of time 210 can be considered as a "treatment period", the second period of time 212 can be considered as a "withdrawal period". The optical treatment can be terminated after the second period of time 212 (not depicted here). Alternatively or in addition, the same type of optical treatment can be resumed or a different type of visual treatment can be applied to both eyes of the person during one or more subsequent periods of time 214.

In the exemplary embodiment of Figure 2B, the spectacle lenses 114, 114' exhibit a different optical treatment for both eyes of the person during a first period of time 210. During a second period of time 212, both spectacle lenses 114, 114' still exhibit a different optical treatment for both eyes of the person, which, however, differs from the optical treatment for both eyes of the person during the first period of time 210. As particularly depicted in Figure 2B, the treatment for the left eye can alter between subsequent periods of time 210, 212. Again, the first period of time 210 can be considered as a "treatment period", while the second period of time 212 can be considered as a "withdrawal period". The optical treatment can be terminated after the second period of time 212 (not depicted here). Alternatively or in addition, the same different types of optical treatment can be resumed or a still further different type of optical treatment can be applied individually to each eye of the person during one or more subsequent periods of time 214,.

Particularly owing to the switching of each electro-optical lens 120, 120', the refractive values of each spectacle lens 114, 114' can be determined for a particular period of time over a wide range. The duration of each period of time 210, 212, 214 can range between 1 second to 2 years, preferably 10 seconds to 1 year, more preferred 1 minute to 6 months, in particular 10 minutes to 1 months, and can, in general, be defined in an experimental manner.

Figure 3 illustrates further exemplary embodiments of a spectacle lens 114, which is designed in accordance with the present invention. As schematically depicted there, the spectacle lens 114 is an electro-optical lens 120, which is divided into four zones denoted by numbers "1", "2", "3" and "4", which can individually be addressed in order to provide a different optical treatment for the corresponding eye of the person during each period of time 210, 212, 214. Again, the first period of time 210 can be considered as a "treatment period", while the second period of time 212 can be considered as a "withdrawal period". In the one or more subsequent periods of time 214, the same type of optical treatment can be resumed or a different type of optical treatment can be applied to the corresponding eye of the person.

Various further embodiments apart from the exemplary embodiments as schematically illustrated in Figures 2A, 2B and 3 are conceivable.

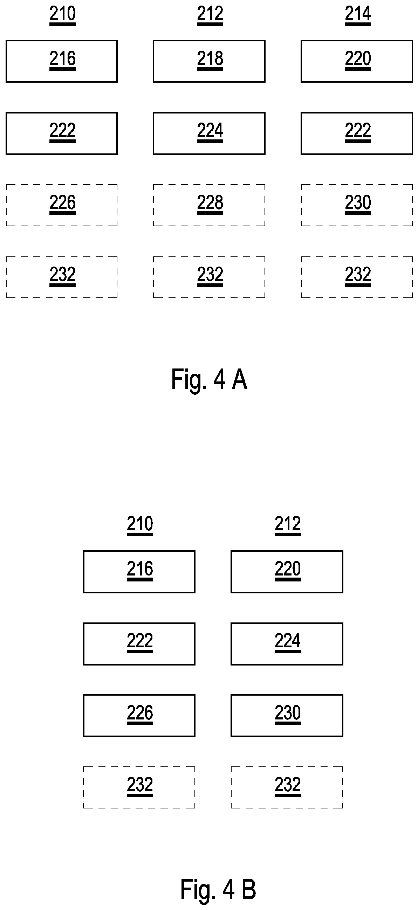

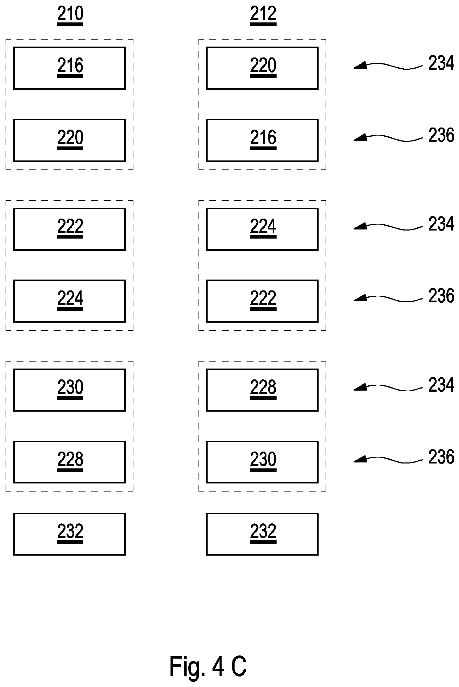

Figure 4 illustrates three exemplary treatment schemes demonstrating the method and the corresponding computer program for controlling the progression of myopia in the at least one eye of the person according to the present invention. Herein, the administration of each exemplary embodiment has been adapted from available studies or clinical trials, which show positive efficacies in managing myopia progression when used as a standalone treatment according to the prior art, in particular selected from
- Jiang Y, Zhu Z, Tan X, Kong X, Zhong H, Zhang J, Xiong R, Yuan Y, Zeng J, Morgan IG, He M, Effect of Repeated Low-Level Red-Light Therapy for Myopia Control in Children: A Multicenter Randomized Controlled Trial, Ophthalmology. 2022;1 29(5):509-519;
- Torii, Hidemasa, Kiwako Mori, Takashi Okano, Shinichiro Kondo, Hao-Yung Yang, Erisa Yotsukura, Akiko Hanyuda, Mamoru Ogawa, Kazuno Negishi, Toshihide Kurihara, and Kazuo Tsubota. 2022. Short-Term Exposure to Violet Light Emitted from Eyeglass Frames in Myopic Children: A Randomized Pilot Clinical Trial, Journal of Clinical Medicine 11, no. 20: 6000;
- Chia A. et al., Atropine for the Treatment of Childhood Myopia: Safety and Efficacy of 0.5%, 0.1%, and 0.01% Doses (Atropine for the Treatment of Myopia 2), Ophthalmology 2012; 119:347-354;
- Yam JC. et al. (LAMP), Low-Concentration Atropine for Myopia Progression Study: A Randomized, Double-Blinded, Placebo-Controlled Trial of 0.05%, 0.025%, and 0.01% Atropine Eye Drops in Myopia Control, Ophthalmology 2019; 126:113-124;
- Edwards MH, Li RW, Lam CS, Lew JK, Yu BS, The Hong Kong progressive lens myopia control study: study design and main findings, Invest Ophthalmol Vis Sci. 2002 Sep; 43(9):2852-8;
- Lam CSY, Tang WC, Tse DY, Lee RPK, Chun RKM, Hasegawa K, Qi H, Hatanaka T, To CH, Defocus Incorporated Multiple Segments (DIMS) spectacle lenses slow myopia progression: a 2-year randomised clinical trial, Br J Ophthalmol. 2020 Mar; 104(3):363-368;
- Bao J, Yang A, Huang Y, et al, One-year myopia control efficacy of spectacle lenses with aspherical lenslets, British Journal of Ophthalmology 2022; 106:1171-1176;
- Rappon J, Chung C, Young G, Hunt C, Neitz J, Neitz M, Chalberg T, Control of myopia using diffusion optics spectacle lenses: 12-month results of a randomised controlled, efficacy and safety study (CYPRESS), Br J Ophthalmol. 2022 Sep 1:bjophthalmol-2021-321005; and
- Pucker, Andrew D. OD, PhD, FAAO1*; Gawne, Timothy J. PhD, FAAO1, Fighting Myopia with Intermittent Nearwork Breaks: 20 Seconds Every 20 Minutes Might Not Be Enough Time, Optometry and Vision Science 100(1): p 31-32, January 2023.

A period time of time of 12 months has been selected exemplarily for the first period of time 210 as the "treatment period", the second period of time 212 as the "withdrawal period", and, if applicable, for the subsequent period of time 214, from these findings.

For the optical treatment, a treatment of one eye or both eyes of the person may, preferably, comprise applying
- a peripheral contrast 216;
- a contrast reduction 218; or
- an accommodation defocus signal 220,
particularly by using the spectacle lens 114, 114', especially the electro-optical lens 120, 120', or, as an alternative, a contact lens, for each eye of the person to be treated. For details of the respective treatments, reference can be made to Lam C.S.Y. et al., Bao J., et al., Edwards M.H., et al., and Rappon J., et al. as cited above.

For the radiation treatment, one eye or both eyes of the person are irradiated by using light of a selected wavelength range, wherein the least one characteristic parameter of the radiation treatment is selected from a parameter related to the wavelength range. Particularly, at least one of the following radiation treatments are preferred:
- a red-light treatment 222, especially having a wavelength of 650 nm at an illuminance level of approximately 1600 lux and a power of 0.29 mW for a pupil size of the person of 4 mm, to be administered for 3 minutes per session, twice daily separated by a minimum time interval of 4 hours, 5 days per week as demonstrated by Jiang Y., et al., see above, to be effective for reducing the progression of myopia; or
- a violet-light treatment 224, especially having a wavelength of 360 nm to 400 nm and an intensity of 310 µW/cm², to be administered for 3 hours per day and 5 days per week, as proposed by Torii et al., see above.

For the pharmaceutical treatment, a dose of atropine is applied to one eye or both eyes of the person; however using a different type of ophthalmic medication, such as cyclopentolate, may also be feasible. As generally known, the effect of myopia suppression and the rebound effect of an atropine treatment depends on the concentration of the atropine, wherein a higher concentration of atropine, generally leads to a higher reduction of myopia progression but also to a higher rebound effect. Accordingly, the following doses, which have been proved to work by Chia A., et al. and Yam J.C. et al., see above, are selected to be used in these exemplary embodiments:
- a first dose 226, especially comprising 0.5% atropine drops once nightly with both eyes;
- a second dose 228, especially comprising 0.05% atropine drops once nightly with both eyes; or
- a third dose 230, especially comprising 0.01% atropine drops once nightly with both eyes.

For the behavioral treatment, a time control for near-vision working 232 is implemented by a module which is configured to trigger a biofeedback signal to the person, wherein the biofeedback signal is designed to initiate the person to take a break having minimum of 5 minutes every hour from near-vision working, as proposed by Pucker et al., see above. However, using a different type of behavioral treatment, in particular a time control for outdoor activities, may also be feasible.

The exemplary treatment scheme as illustrated in Figure 4A is, in particular, intended to provide a sustainable efficacy of the treatment of the progression of myopia in both eyes of a person. As depicted there, the treatment processes 216, 222, 226, 232, each selected from a different class, are simultaneously applied to both eyes of the person during the first period of time 210. In Figures 4A and 4B, the particularly preferred treatment processes are indicated in solid lines, while preferred treatment processes are indicated in dashed lines. During the subsequent second period of time 212, the further treatment processes 218, 224, 228, 232, each of which is selected from the same class, are simultaneously applied to both eyes the person, wherein three of the further treatment processes applied during the second period of time 212 differ here from the corresponding preceding treatment process as applied during the first period of time 210 by a value of a characteristic parameter by which the respective treatment processes is determined.

As further depicted in Figure 4A, this exemplary treatment scheme is continued here with the still further treatment processes 220, 222, 230, 232, each of which is again selected from the same class, to be simultaneously applied to both eyes of the person for a subsequent period of time 214, wherein three of the still further treatment processes applied during the subsequent period of time 214 differ here from the corresponding preceding treatment process as applied during the second period of time 212 again by a value of a characteristic parameter by which the respective treatment processes is determined.

The further exemplary treatment scheme as illustrated in Figure 4B is, in particular, intended to address a rebound effect which may occur as a consequence of terminating the treatment of the progression of myopia in both eyes of the person. As depicted there, the treatment processes 216, 222, 226, 232, each selected from a different class, are simultaneously applied to both eyes of the person during the first period of time 210. During the subsequent second period of time 212, the further treatment processes 220, 224, 230, 232, each of which is selected from the same class, are simultaneously applied to both eyes the person, wherein again three of the further treatment processes applied during the second period of time 212 differ here from the corresponding preceding treatment process as applied during the first period of time 210 by a value of a characteristic parameter by which the respective treatment processes is determined.

The further exemplary treatment scheme as illustrated in Figure 4C is, again, particularly intended to address the rebound effect that may occur as a consequence of terminating the treatment of the progression of myopia in both eyes of a person. As depicted there, the treatment processes 216, 222, 230, 232, each selected from a different class, are applied to a first eye 234 of the person during the first period of time 210, while the treatment processes 220, 224, 228, 232, each again selected from a different class but having a different value of the corresponding characteristic parameter, are, simultaneously, applied to a second eye 236 of the person during the same first period of time 210. During the subsequent second period of time 212, the treatment processes 216, 222, 230, 232, which have been applied to the first eye 234 of the person of the person during the first period of time 210, are now applied to the second eye 236 of the person. Similarly, the different treatment processes 220, 224, 228, 232, which have been applied to the second eye 236 of the person of the person during the first period of time 210, are now applied to the first eye 234 of the person.

Alternatively or in addition to the exemplary treatment schemes as illustrated in Figures 4A to 4C, further kinds of treatment schemes may also be feasible.

### List of Reference Signs

- 110: system for controlling a progression of myopia in at least one eye of a person
- 112: spectacle frame
- 114, 114': spectacle lens
- 116: processing device
- 118: smartphone
- 120, 120': electro-optical lens
- 122, 122': light source
- 124, 124': light-emitting diode
- 126: illumination sensor
- 128, 128': dispenser
- 130, 130': biofeedback device
- 132: distance sensor
- 134: eye tracker
- 136: screen
- 138: graphic presentation of a treatment scheme
- 140: diagram showing results of a treatment process on progression of myopia
- 142: loudspeaker
- 144: virtual keyboard
- 146: microphone
- 148: front camera
- 210: first period of time
- 212: second period of time
- 214: subsequent period of time
- 216: optical treatment by application of a peripheral contrast
- 218: optical treatment by application of a contrast reduction
- 220: optical treatment by application of accommodation defocus signal
- 222: red-light radiation treatment
- 224: violet-light radiation treatment
- 226: pharmaceutical treatment by application of a first dose of atropine
- 228: pharmaceutical treatment by application of a second dose of atropine
- 230: pharmaceutical treatment by application of a third dose of atropine
- 232: behavioral treatment comprising a recommendation to the person to reduce near-vison working
- 234: first eye
- 236: second eye

## Claims

1. A method for controlling a progression of myopia in at least one eye of a person, the method comprising the following steps:
- applying a treatment process during a period of time, wherein the treatment process is designed for reducing the progression of myopia in at least one eye of a person, wherein the treatment process is determined by at least one characteristic parameter, wherein the treatment process is selected from a class of treatment processes comprising:
∘ an optical treatment of the at least one eye of the person;
∘ a radiation treatment of the at least one eye of the person;
∘ a pharmaceutical treatment of the at least one eye of the person; and
∘ a behavioral treatment of the person,
and
- determining at least one result of the treatment process on the progression of the myopia in the at least one eye of the person,
wherein at least two treatment processes, each selected from a different class, are simultaneously applied to the person during a first period of time (210).

2. The method according to the preceding claim, wherein, during at least one subsequent period of time (212, 214), at least two further treatment processes selected from the same two classes are simultaneously applied to the person, wherein at least one of the further treatment processes differs from at least one preceding treatment process by a value of the least one characteristic parameter by which the treatment process is determined.

3. A computer program for controlling a progression of myopia in at least one eye of a person, wherein the computer program comprises instructions which, when the program is executed by a computer, cause the computer to carry out the following steps:
- applying a treatment process during a period of time, wherein the treatment process is designed for reducing the progression of myopia in at least one eye of a person, wherein the treatment process is determined by at least one characteristic parameter, wherein the treatment process is selected from a class of treatment processes comprising:
∘ an optical treatment of the at least one eye of the person;
∘ a radiation treatment of the at least one eye of the person;
∘ a pharmaceutical treatment of the at least one eye of the person; and
∘ a behavioral treatment of the person,
and
- determining at least one result of the treatment process on the progression of the myopia in the at least one eye of the person,
wherein at least two treatment processes, each selected from a different class, are simultaneously applied to the person during a first period of time (210).

4. The computer program according to the preceding claim, wherein, during at least one subsequent period of time (212, 214), at least two further treatment processes selected from the same two classes are simultaneously applied to the person, wherein at least one of the further treatment processes differs from at least one preceding treatment process by a value of the least one characteristic parameter by which the treatment process is determined.

5. The computer program according to the preceding claim, wherein the value of at least one particular characteristic parameter by which a particular treatment process is determined differs from the value of the least one particular characteristic parameter by which a preceding treatment process selected from the same class is determined.

6. The computer program according to any one of the preceding claims, wherein at least two of the optical treatment, the radiation treatment, or the pharmaceutical treatment are provided simultaneously to both eyes of the person, wherein the value of at least one particular characteristic parameter by which a particular treatment process to a particular eye of the person is determined differs from or is identical with the value of the least one particular characteristic parameter by which a preceding treatment process to the other of the particular eye of the person selected from the same class is determined.

7. The computer program according to the preceding claim, wherein the least one characteristic parameter of the optical treatment is selected from a parameter related to at least one of:
- a peripheral contrast (216);
- a color;
- a contrast reduction (218); or
- a accommodation defocus signal (220)
provided via at least one spectacle lens (114, 114') to the at least one eye of the person.

8. The computer program according to any one of the preceding claims, wherein the at least one characteristic parameter of the optical treatment is selected individually for at least one zone in the at least one spectacle lens (114, 114') applied for the optical treatment of the at least one eye of the person.

9. A system (110) for controlling a progression of myopia in at least one eye of a person, the system comprising:
- a spectacle frame (112) comprising a pair of spectacle lenses (114, 114') and at least one of the spectacle lenses (114, 114') incorporated by the spectacle frame (112), wherein the spectacle frame (114) or at least one of the spectacle lenses (114, 114') are configured for applying a treatment process during a period of time, wherein the treatment process is designed for reducing the progression of myopia in at least one eye of a person, wherein the treatment process is determined by at least one characteristic parameter, wherein the treatment process is selected from a class of treatment processes comprising:
∘ an optical treatment of the at least one eye of the person;
∘ a radiation treatment of the at least one eye of the person;
∘ a pharmaceutical treatment of the at least one eye of the person; and
∘ a behavioral treatment of the person,
and
- a processing device (116) configured for determining at least one result of the treatment process on the progression of the myopia in the at least one eye of the person,
wherein the spectacle frame (112) or at least one of the spectacle lenses (114, 114') are configured for applying at least two treatment processes, each selected from a different class, simultaneously to the person during a first period of time (210).

10. The system (110) according to the preceding claim, wherein the spectacle frame (112) or at least one of the spectacle lenses (114, 114') are configured for applying
- at least one of the optical treatment, the radiation treatment, or the pharmaceutical treatment simultaneously to both eyes of the person;
- at least one of a different optical treatment, a different radiation treatment, or a different pharmaceutical treatment to each eye of the person.

11. The system (110) according to any one of the preceding system claims, wherein the spectacle lenses (114, 114') are designed to provide the optical treatment by application of at least one of:
- a peripheral contrast (216);
- a color;
- a contrast reduction (218); or
- an accommodation defocus signal (220)
to the at least one eye of the person.

12. The system (110) according to any one of the preceding system claims, wherein at least one of the spectacle lenses (114, 114') is or comprises an electro-optical lens (120, 120').

13. The system (110) according to the preceding claim, wherein
- at least two zones in the at least one electro-optical lens (120, 120') are individually addressable; or
- the electro-optical lens (120, 120') is configured for irradiating the at least one eye of the person by light of a selected wavelength range.

14. The system (110) according to any one of the two preceding claims, wherein the spectacle frame further comprises at least one of
- a light source (122, 122'), preferably at least one light-emitting diode (124, 124');
- an illumination sensor (128);
- a dispenser (128, 128') configured for applying a dose of at least one ophthalmic medication to the at least one eye of the person;
- a receptacle configured for storing a portion of the ophthalmic medication;
- a biofeedback device (130), preferably a beeping element or a buzzing element, configured for generating the at least one biofeedback signal and for providing the at least one biofeedback signal to an attention of the person;
- a distance sensor (132) configured for recording at least one distance of the at least one eye of the person in viewing direction to the at one spectacle lens (114, 114');
- an eye tracker (134, 134') configured for recording at least one movement of the at least one eye of the person.

15. The system (110) according to any one of the preceding system claims, wherein the processing device (116) is further configured for at least one of
- selecting the period of time (210) and the at least one subsequent period of time (212, 214);
- determining the at least one value for the at least one characteristic parameter;
- determining the at least one value of the least one characteristic parameter in the at least one subsequent period of time (212, 214) by using the at least one result of the at least two treatment processes in at least one preceding period of time;
- providing communication between at least two parts of the system (110);
- generating at least one signal for modulating the at least one electro-optical lens (120, 120');
- switching the at least one light source (122, 122', preferably the at least one light-emitting diode (124, 124');
- controlling the at least one dispenser (128, 128');
- recording at least one signal received from the at least one eye-tracker (134, 134').
